# EUROPEAN PATENT SPECIFICATION

(11) **EP 3 432 736 B1**
(45) Date of publication and mention of the grant of the patent: **22.01.2020**
(21) Application number: 17712957.4
(22) Date of filing: 21.03.2017
(51) Int. Cl.: H05B 1/02, H05B 3/26, A24F 47/00, B67D 7/00, B65D 1/44

(54) **ELECTRONIC VAPING DEVICE**
ELEKTRONISCHE VAPENVORRICHTUNG
DISPOSITIF ÉLECTRONIQUE DE VAPOTAGE

(30) Priority: 21.03.2016 US 201615075588
(43) Date of publication of application: 30.01.2019
(73) Proprietor: Philip Morris Products S.A., 2000 Neuchâtel (CH)
(72) Inventor: LIPOWICZ, Peter, Midlothian, Virginia 23112 (US)
(74) Representative: Lupini, Stephen Antony
(86) International application number: PCT/EP2017/056732
(87) International publication number: WO 2017/162691

(56) References cited:
- EP-A1- 2 316 286
- EP-A1- 2 340 729
- WO-A1-2016/014652
- DE-U1-202015 006 397
- US-A- 5 573 692
- US-A1- 2014 060 554
- US-A1- 2015 351 456

## Description

The present disclosure relates to an electronic vaping or e-vaping device configured to deliver a pre-vapor formulation to a vaporizer.

An electronic vaping device includes a heater element which vaporizes a pre-vapor formulation to produce a "vapor." The heater element may include a resistive heater coil, with a wick extending there through.

US 2014/060554 A1 describes an electronic smoking article comprising a rod-like shell having a distal end and a mouth-end with a mouth opening. The smoking article further comprising a microheater, a reservoir for holding an aerosol precursor composition and a transport element which extends from the reservoir and into proximity with the microheater such that the aerosol precursor composition can be delivered to the microheater for aerosolization. The formed aerosol is then drawn by a user through the mouth-end of the smoking article.

At least one example embodiment relates to an electronic vaping device.

According to an aspect of the present disclosure, there is provided an electronic vaping device including a housing extending in a longitudinal direction, the housing having a tip end and a mouth-end, the tip end being closed and the mouth-end having an opening therein, a planar heater contained in the housing, a heater support configured to support the planar heater, the heater support comprising a disc-shaped body that friction fits with an inner surface of the housing, and a tubular body on which the heater is mounted, the tubular body extending downstream from the disc-shaped body. The electronic vaping device further including a tank containing a pre-vapor formulation, the tank configured to slide into and out of the opening of the mouth-end of the housing, and a wick extending from the tank. The wick is configured to be in contact with the planar heater when the tank is inserted in the housing.

In some example embodiments, the electronic vaping device includes a mouth-end insert configured to be inserted in the mouth-end of the housing. The mouth-end insert may include at least one outlet.

In some example embodiments, the electronic vaping device includes a stop on an inner surface of the housing, the stop configured to substantially prevent the tank from being inserted too far into the housing.

In some example embodiments, the housing is unitary. The wick may be formed of cellulose. The wick may be monolithic. The tank may include one or more ribs running longitudinally along an outer surface of the tank.

In some example embodiments, the planar heater includes a patterned layer of platinum disposed on a ceramic layer of material. The patterned layer of platinum is configured to be in electrical communication with a power supply through leads electrically connected to the patterned layer of platinum. The power supply may be configured to supply power to the patterned layer of platinum so as to resistively heat the patterned layer of platinum such that the heater may reach a temperature sufficient to vaporize the pre-vapor formulation. The patterned layer of platinum may have a resistivity of about 1 to 6 ohms. The leads may be formed from platinum coated nickel wire. The heater may be in the shape of a polyhedron having a square, triangular, diamond or rectangular shaped base with rounded or sharp corners. The heater may have a square or rectangular base wherein a length and width of the heater are each about 1.5 millimetres to about 4 millimetres and a thickness of the heater is about 0.2 millimetres to about 0.8 millimetres.

In some example embodiments, a glass layer of material may be disposed on the ceramic layer such that the patterned layer of platinum is between the ceramic layer and the glass layer. The ceramic layer may be a first ceramic layer, and a second ceramic layer may be disposed on the first ceramic layer such that the patterned layer of platinum is between the first ceramic layer and the second ceramic layer. The ceramic layer may be formed from alumina, titania, zirconia, yttria, or yttria-stabilized zirconia. The patterned layer of platinum may be about 0.5 micron to about 2 microns thick and may have a width ranging from about 1 micron to about 100 microns.

In at least one example embodiment, the patterned layer of platinum has a sinuous pattern. In other example embodiments, the patterned layer of platinum has a U-shaped pattern.

In some example embodiments, the patterned layer of platinum includes first conductors, second conductors, and at least two heater portions arranged in parallel between the first and second conductors. The heater portions have a higher resistivity than the first and second conductors.

In some example embodiments, the heater includes a first patterned layer of platinum which has a higher resistivity than a second patterned layer of platinum. The first patterned layer of platinum is configured to be in electrical communication with the power source through a first set of leads and the second layer of platinum is configured to be in electrical communication with the power source through a second set of leads.

In some example embodiments, the first patterned layer of platinum is sinuous and the second patterned layer of platinum is U-shaped.

In at least one example embodiment, the ceramic layer of material includes at least one groove in a surface thereof. The groove is configured to direct a flow of the pre-vapor formulation from the wick toward a portion of the heater which reaches a temperature sufficient to vaporize pre-vapor formulation.

In some example embodiments, the ceramic layer of material includes at least one through-hole extending through a thickness of the ceramic layer. The at least one through-hole exposes portions of the patterned layer of platinum. The through-hole is configured to direct a flow of the pre-vapor formulation from the wick toward a portion of the heater. The ceramic layer of material may be porous. The ceramic layer of material may include at least one bump. The bump is configured to direct a flow of the pre-vapor formulation from the wick toward a portion of the heater.

In some example embodiments, the patterned layer of platinum includes first and second conductors and a heater portion arranged between the first and second conductors. The first and second conductors each have a thickness of about 20 microns and the heater portion has a thickness of about 2 microns. The patterned layer of platinum may include a gold coating on an outer surface thereof. The patterned layer of platinum may be configured to concentrate heat at a tip thereof. The tip of the heater is thermally isolated from the remainder of the heater. The electronic vaping device may have a uniform diameter of less than about 10 millimetres.

In some example embodiments, the electronic vaping device includes control circuitry including a sensor. The sensor is configured to sense a change in pressure. The electronic vaping device may also include at least one light emitting diode at the tip end.

The various features and advantages of the non-limiting embodiments herein may become more apparent upon review of the detailed description in conjunction with the accompanying drawings. The accompanying drawings are merely provided for illustrative purposes and should not be interpreted to limit the scope of the claims. The accompanying drawings are not to be considered as drawn to scale unless explicitly noted. For purposes of clarity, various dimensions of the drawings may have been exaggerated.
FIG. 1 is a side view of an electronic vaping device according to an example embodiment.
FIG. 2 is an illustration of an electronic vaping device having a transparent housing.
FIG. 3 is perspective view of a heater and support according to at least one example embodiment.
FIG. 4 is an illustration of a tank being inserted into a mouth-end of an electronic vaping device according to at least one example embodiment.
FIG. 5 is an enlarged view of a tank according to some example embodiments.
FIG. 6 is an enlarged view of a wick in contact with a heater according to at least one example embodiment.
FIG. 7 is a cross-sectional view of an outer housing along line VII-VII of FIG. 2 according to at least one example embodiment.
FIGS. 8A and 8B are cross-sectional views of a heater of an electronic vaping device according to at least one example embodiment.
FIG. 9 is a power supply graph for a heater.
FIGS. 10A-10D are cross-sectional views of a heater of an electronic vaping device.
FIGS. 11A-11D are cross-sectional views of a heater of an electronic vaping device.
FIGS. 12A-12B are cross-sectional views of a heater of an electronic vaping device.
FIGS. 13A-13B are cross-sectional views of a heater of an electronic vaping device.
FIGS. 14A-14C are cross-sectional views of a heater of an electronic vaping device.

Some detailed example embodiments are disclosed herein. However, specific structural and functional details disclosed herein are merely representative for purposes of describing example embodiments. Example embodiments may, however, be embodied in many alternate forms and should not be construed as limited to only the example embodiments set forth herein.

Accordingly, while example embodiments are capable of various modifications and alternative forms, example embodiments thereof are shown by way of example in the drawings and will herein be described in detail. It should be understood, however, that there is no intent to limit example embodiments to the particular forms disclosed, but to the contrary, example embodiments are to cover all modifications, equivalents, and alternatives falling within the scope of example embodiments. Like numbers refer to like elements throughout the description of the figures.

It should be understood that when an element or layer is referred to as being "on," "connected to," "coupled to," or "covering" another element or layer, it may be directly on, connected to, coupled to, or covering the other element or layer or intervening elements or layers may be present. In contrast, when an element is referred to as being "directly on," "directly connected to," or "directly coupled to" another element or layer, there are no intervening elements or layers present. Like numbers refer to like elements throughout the specification.

It should be understood that, although the terms first, second, third, and so forth may be used herein to describe various elements, components, regions, layers or sections, these elements, components, regions, layers, or sections should not be limited by these terms. These terms are only used to distinguish one element, component, region, layer, or section from another element, component, region, layer, or section. Therefore, a first element, component, region, layer, or section discussed below could be termed a second element, component, region, layer, or section without departing from the teachings of example embodiments.

Spatially relative terms (for example, "beneath," "below," "lower," "above," "upper," and the like) may be used herein for ease of description to describe one element or feature's relationship to another element or feature as illustrated in the figures. It should be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Therefore, the term "below" may encompass both an orientation of above and below. The device may be otherwise oriented (rotated 90 degrees or at other orientations) and the spatially relative descriptors used herein interpreted accordingly.

The terminology used herein is for the purpose of describing various example embodiments only and is not intended to be limiting of example embodiments. As used herein, the singular forms "a," "an," and "the" are intended to include the plural forms as well, unless the context clearly indicates otherwise. It will be further understood that the terms "includes," "including," "comprises," and "comprising," when used in this specification, specify the presence of stated features, integers, steps, operations, elements, or components, but do not preclude the presence or addition of one or more other features, integers, steps, operations, elements, components, or groups thereof.

Example embodiments are described herein with reference to cross-sectional illustrations that are schematic illustrations of idealized embodiments (and intermediate structures) of example embodiments. As such, variations from the shapes of the illustrations as a result, for example, of manufacturing techniques or tolerances, are to be expected. Therefore, example embodiments should not be construed as limited to the shapes of regions illustrated herein but are to include deviations in shapes that result, for example, from manufacturing.

Unless otherwise defined, all terms (including technical and scientific terms) used herein have the same meaning as commonly understood by one of ordinary skill in the art to which example embodiments belong. It will be further understood that terms, including those defined in commonly used dictionaries, should be interpreted as having a meaning that is consistent with their meaning in the context of the relevant art and will not be interpreted in an idealized or overly formal sense unless expressly so defined herein.

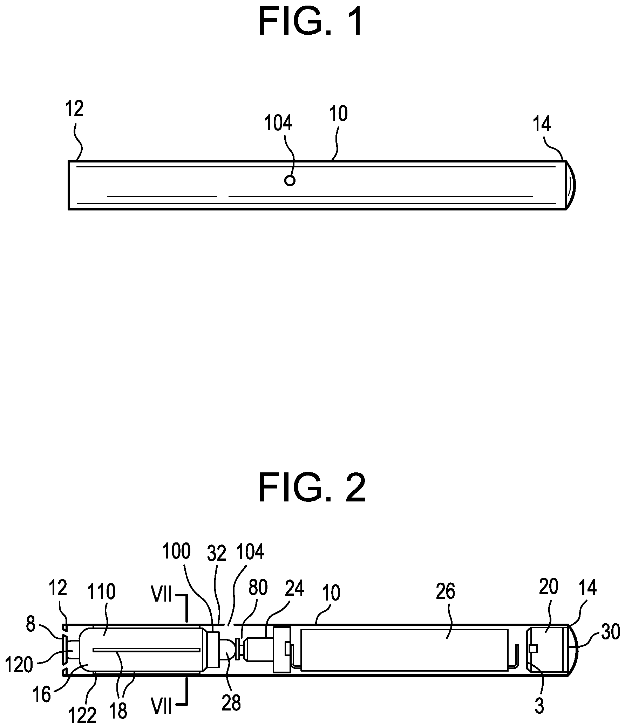

In at least one example embodiment, as shown in FIGS. 1-2, an electronic vaping device 10 has a mouth-end 12 and a tip end 14. An outer housing 32 extends in a longitudinal direction from the mouth-end 12 to the tip end 14. The mouth-end 12 may include an opening 5 therein.

The outer housing 32 may have a generally cylindrical cross-section. In other example embodiments, the outer housing 32 may have a generally triangular cross-section or square cross-section. In some example embodiments, the housing 32 may have a greater circumference or dimensions at the tip end 14 than at a mouth-end 12 of the electronic vaping device 10 or vice versa. In at least one example embodiment, the housing 32 is a single, unitary housing. In other example embodiments, the housing 32 may include two or more pieces.

In some example embodiments, as shown in FIG. 2, the electronic vaping device 10 includes a mouth-end insert 8 configured to be inserted in the opening 5 of the mouth-end 12 of the housing 32. The mouth-end insert 8 may include at least one outlet.

As shown in FIG. 2, in at least one example embodiment, the housing 32 contains a tank 16. The tank 16 contains a pre-vapor formulation and has an opening 113 at an upstream end 100. A wick 28 extends from the upstream end 100 of the tank 16.

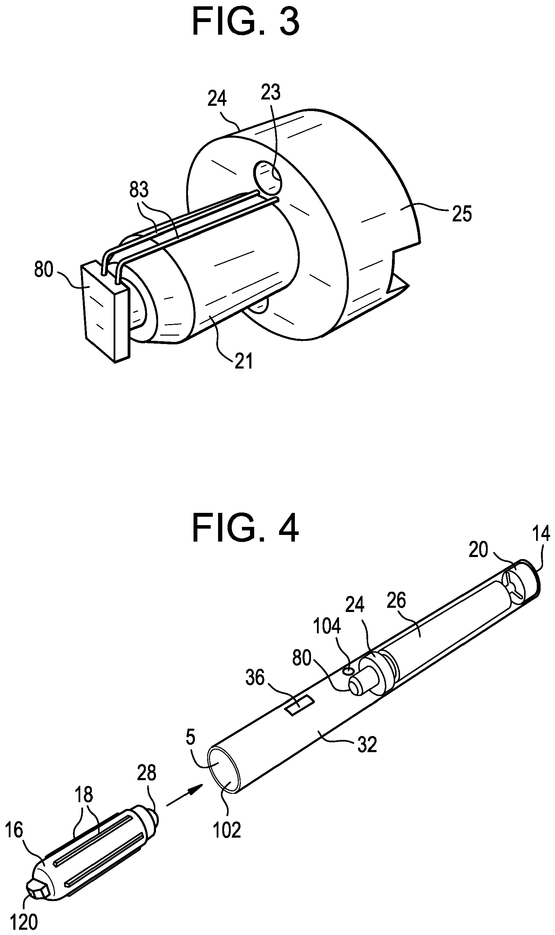

In at least one example embodiment, when the tank 16 is inserted in the housing 32, the wick 28 contacts a heater 80 that is supported by a support 24 (shown in FIGS. 2-3). As shown in FIGS. 3-4, electrical leads 83 electrically connect the heater 80 with a power supply 26 and control circuitry 20.

In some example embodiments, the control circuitry 20 may include a sensor 3, such as at least one of a negative-pressure sensor and a microelectromechanical (MEMS) sensor. At least one light emitting diode (LED) 30 (shown in FIG. 2) may be positioned at the tip end 14, such that the LED 30 lights up when the electronic vaping device 10 is being recharged, vaped, or both.

The pre-vapor formulation contained in the tank 16 may be a material or combination of materials that may be transformed into a vapor. For example, the pre-vapor formulation may be at least one of a liquid, solid or gel formulation including, but not limited to, water, beads, solvents, active ingredients, ethanol, plant extracts, natural or artificial flavors, vapor formers such as glycerin and propylene glycol, and combinations thereof.

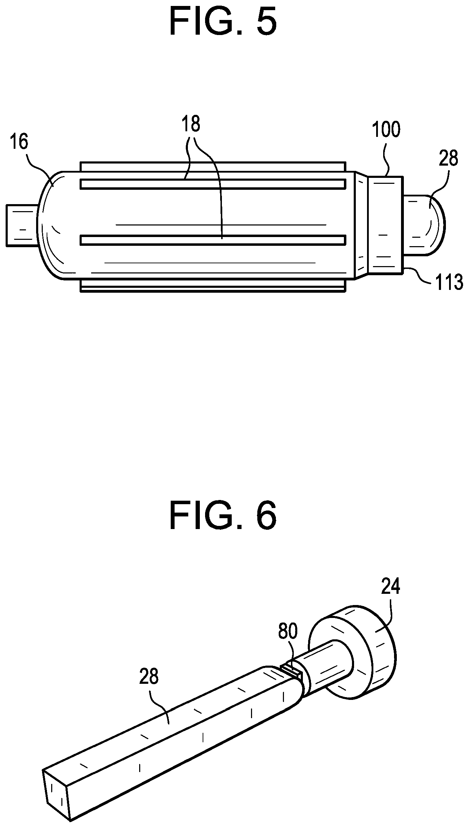

In at least one example embodiment, as shown in FIGS. 5-6, the wick 28 is a monolithic body formed of cellulose. Since cellulose swells in contact with the pre-vapor formulation, the wick 28 also seals the opening 113 in the tank 16 so as to substantially prevent or reduce leakage of the pre-vapor formulation from the tank 16 during storage, vaping, or both.

Moreover, since the wick 28 seals the opening 113 of the tank 16, the pre-vapor formulation does not contact the heater 80. Since the heater 80 includes metal, substantially preventing the pre-vapor formulation from contacting the heater 80 during storage may prevent or abate chemical reactions between the metal and the pre-vapor formulation that may cause the pre-vapor formulation to be unstable.

In some example embodiments, the tank 16 may include a plurality of ribs 18 running longitudinally along an outer surface 110 of the tank 16. The ribs 18 space remaining portions of the tank 16 from an inner surface 102 of the outer housing 32, such that air may flow along the tank 16 between the tank 16 and the inner surface 102 of the outer housing 32 during vaping. Air may be drawn into the electronic vaping device 10 via one or more air inlets 104 located upstream of the tank 16.

The tank 16 may be removable and replaceable once the pre-vapor formulation is depleted. To insert the tank, as shown in FIG. 4, the tank 16 may be pushed into the mouth-end 12 of the housing 32. To facilitate removal of the tank 16 from the housing 32, a grip 120 may be formed on a downstream end 122 of the tank 16.

In at least one example embodiment, the tank 16 is formed of at least one of a plastic and glass. Suitable plastics include polyethylene terephthalate, polyethylene, polyester, cyclic olefin copolymer, nylon, and polypropylene. The use of plastics, glass, or both, to form the tank 16 aids in maintaining the stability of the pre-vapor formulation because the pre-vapor formulation is substantially prevented from contacting metals, reacting with metals, or both.

Moreover, since the pre-vapor formulation is contained in the tank 16 located downstream of the heater 80, electrical leads 83 do not extend through the tank 16 and do not contact the pre-vapor formulation to further prevent or abate reaction of the pre-vapor formulation with any metals.

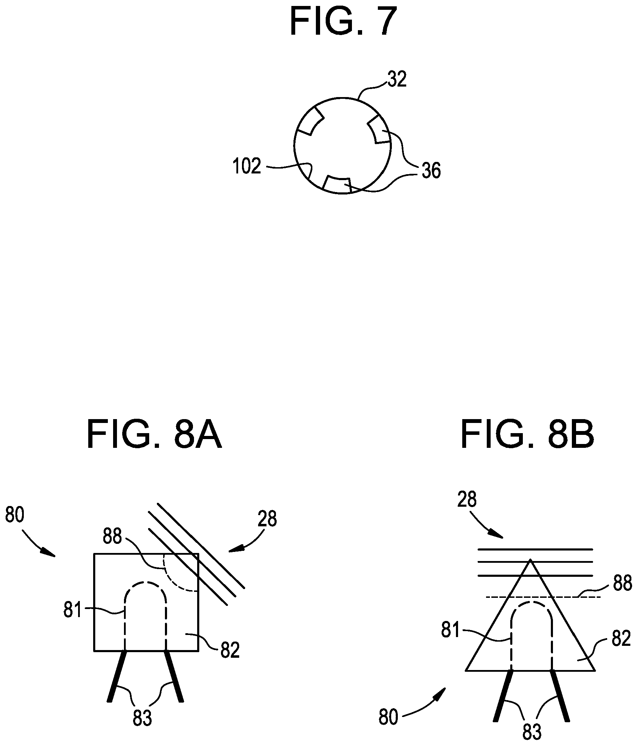

As shown in FIGS. 4 and 7, in at least one example embodiment, at least one stop 36 may be formed on the inner surface 102 of the outer housing 32. The at least one stop 36 may be a ridge or bump on the inner surface 102. The at least one stop 36 is configured to substantially prevent insertion of the tank 16 too far into the outer housing 32, so as to substantially avoid or mitigate damage to the heater 80. The at least one stop 36 is positioned so that that after insertion of the tank 16 in the housing 32, the ribs 18 abut the stop 36 and the wick 28 contacts the heater 80.

In at least one example embodiment, as shown in FIG. 3, the support 24 includes a disc-shaped body 25 that friction fits with the inner surface 102 of the outer housing 32. The disc-shaped body 25 may form a seal with the inner surface 102 of the outer housing 32. A tubular body 21 extends downstream from the disc-shaped body 25, such that the support 24 is generally T-shaped in cross-section. The tubular body 21 supports the heater 80 so as to reduce at least one of bending and breaking of the heater 80 during insertion of the tank 16, during shipping, during vaping, and combinations thereof. The electrical leads 83 extend from the heater 80, along the tubular body 21 and through one or more openings 23 in the disc-shaped body 25.

In at least one example embodiment, the electrical leads 83 connect the heater 80 to the power supply 26 and the control circuitry 20.

In at least one example embodiment, as shown in FIGS. 2 and 4, the power supply 26 may include a battery arranged in the electronic vaping device 10. The power supply 26 may be a Lithium-ion battery or one of its variants, for example a Lithium-ion polymer battery. Alternatively, the power supply 26 may be a nickel-metal hydride battery, a nickel cadmium battery, a lithium-manganese battery, a lithium-cobalt battery or a fuel cell. The electronic vaping device 10 may be usable by an adult vaper until the energy in the power supply 26 is depleted or in the case of lithium polymer battery, a minimum voltage cut-off level is achieved.

Further, the power supply 26 may be rechargeable and may include circuitry configured to allow the battery to be chargeable by an external charging device. To recharge the electronic vaping device 10, an USB charger or other suitable charger assembly may be used.

Further, the control circuit 20 may supply power to the heater 80 responsive to the sensor. In one example embodiment, the control circuit 20 may include a maximum, time-period limiter. In another example embodiment, the control circuit 20 may include a manually operable switch. The time-period of the electric current supply to the heater 80 may be pre-set depending on the amount of pre-vapor formulation desired to be vaporized. In yet another example embodiment, the control circuit 20 may supply power to the heater 80 as long as the sensor 3 detects a pressure drop.

When activated, the heater 80 may heat a portion of the wick 28 for less than about 10 seconds. Therefore, the power cycle may range in period from about 2 seconds to about 10 seconds (for example, about 3 seconds to about 9 seconds, about 4 seconds to about 8 seconds or about 5 seconds to about 7 seconds).

In at least one example embodiment, as shown in FIGS. 2 and 3, the heater 80 is a planar heater that contacts at least a portion of the wick 28, but is not intertwined or wrapped around the wick 28.

Manufacture of the electronic vaping device 10 is simple and may be automated since the heater 80 and wick 28 need not be intertwined. Moreover, since the tank 16 is removable, the overall structure of the electronic vaping device 10 is simpler and includes fewer parts as compared to electronic vaping devices having an annular reservoir and a coil heater wrapped around a wick.

FIGS. 8A and 8B each illustrate at least one example embodiment of the heater 80 according to some example embodiments. As shown, the heater 80 may include a patterned layer of platinum 81 disposed on a ceramic layer 82 of material. Electrical leads (leads) 83 are electrically connected to the patterned layer of platinum 81 such that the patterned layer of platinum 81 may be electrically connected to the power source (not shown).

In at least one example embodiment, the ceramic layer 82 may be formed from alumina, titania, zirconia, yttria, or yttria-stabilized zirconia or other suitable material. The ceramic layer of material 82 may be porous such that the pre-vapor formulation may be absorbed by the ceramic layer of material 82.

In some example embodiments, the patterned layer of platinum 81 may include impurities therein or may be a platinum alloy. In an example embodiment, the patterned layer of platinum 81 may include a gold coating on an outer surface thereof.

In at least one example embodiment, the ceramic layer 82 is alumina and the patterned layer of platinum 81 is formed from platinum having a purity of 99 percent or greater. In at least one example embodiment, the layer of platinum 81 may include a platinum alloy including up to 20 percent rhodium so as to achieve a lower temperature coefficient of resistance. The patterned layer of platinum 81 may have a temperature coefficient of about 0.0005 to about 0.005 per degree Celsius at about 20 degrees Celsius. The leads 83 may be formed from platinum coated nickel wire, nickel wire, Nichrome wire, stainless steel wire, and combinations thereof.

In at least one example embodiment, the resistance of the patterned layer of platinum 81 may be about 1 ohm to about 6 ohms at room temperature, such that the resistance of the patterned layer of platinum 81 increases as the temperature of the patterned layer of platinum 81 increases. The heater 80 is self-regulating against overdriving or overheating because as the patterned layer of platinum 81 of the heater 80 increases in temperature, the platinum forming the patterned layer increases in resistivity, which tends to lower the heating rate of the patterned layer of platinum 81 when a constant voltage is supplied across the patterned layer of platinum 81.

For a constant voltage, the effect of a decrease in resistance will increase the power supplied to the patterned layer of platinum 81 as P = V²/R wherein P stands for power, V stands for voltage, and R stands for resistance. For example, the resistance of the patterned layer of platinum 81 decreases when the temperature of the patterned layer of platinum 81 decreases. In at least one example embodiment, where the thermal load is what is being heated, decreasing the load may increase the heater temperature and raise the resistance. When the resistance of the patterned layer of platinum decreases (which tends to in and of itself decrease resistive heating), the power supplied through the patterned layer of platinum 81 will increase, which increases resistive heating and thereby causes the heater 80 to be self-regulating. In addition, the current and voltage may be measured by the device to determine the heater temperature.

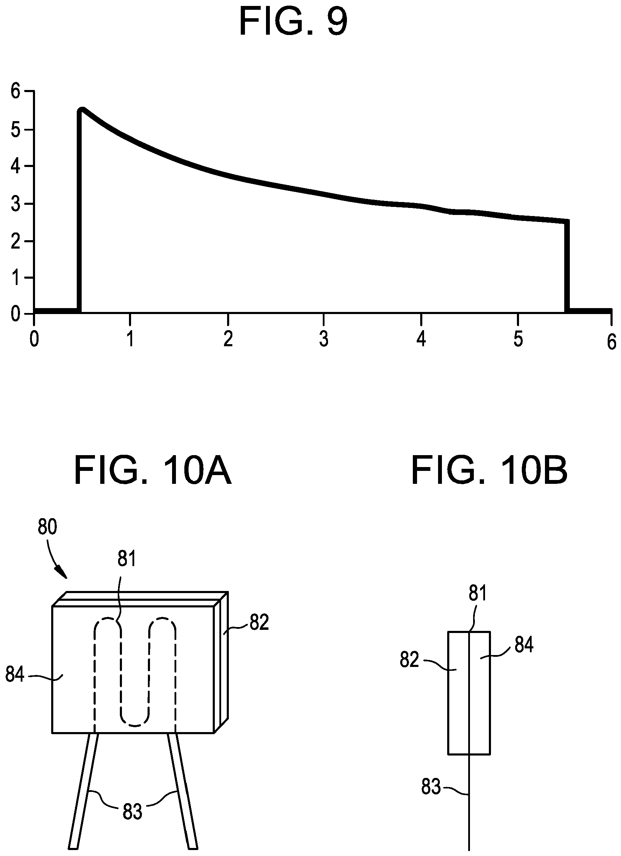

As shown in FIG. 9, an amount of power supplied in Watts (y-axis) to a patterned layer of platinum 81 of the heater 80 is measured against the amount of time in seconds (x-axis) the power is supplied to the patterned layer of platinum 81. In this example embodiment, voltage is supplied across the patterned layer of platinum 81 at a constant level of about 3.7 volts for a heating period of about 5 seconds. The patterned layer of platinum 81 initially has a resistance of about 2.5 ohms at a temperature of about 25 degrees Celsius (room temperature). The power supply is turned on at about 0.5 seconds wherein the low initial resistance of the patterned layer of platinum 81 results in a rapid initial application of power (about 5.5 Watts) to the patterned layer of platinum 81 such that the patterned layer of platinum 81 is rapidly heated. As time progresses, and the patterned layer of platinum 81 increases in resistance, less power is supplied thereto. For example, just before the power supply is turned off at about 5.5 seconds, only about 3 Watts of power is supplied to the patterned layer of platinum 81. At this point, the temperature of the patterned layer of platinum 81 has increased to about 337 degrees Celsius and the resistance of the patterned layer of platinum has increased to about 5.5 ohms.

As shown in the graph shown in FIG. 9, more power is drawn during the beginning portion of the heating period than at the end portion of the heating period. Therefore, the initial application of power may rapidly enhance vapor generation by quickly increasing the temperature of the patterned layer of platinum 81, while power supplied to the patterned layer of platinum 81 is reduced as the temperature of the patterned layer of platinum 81 increases. Therefore, power is saved as the resistance of the patterned layer of platinum increases. The reduction in power requirements may increase the battery life of the power supply 26, and may also allow for power sources with reduced battery capacity or size to be included in the power supply 26 of the electronic vaping device 10.

In at least one example embodiment, the heater 80 is arranged to contact the wick 28, such that the heater 80 may vaporize the pre-vapor formulation through conduction, convection, or both.

In another example embodiment, the heater 80 may be in the shape of a polyhedron, and for example may have a rectangular-shaped, diamond-shaped, or triangular-shaped base, or square shaped base. Corners of the polyhedron may be rounded or sharp. In an example embodiment, the polyhedron shaped heater 80 may have a square or rectangular base wherein a length and width of the heater are each about 1.5 millimetres to about 3 millimetres and a thickness of the heater is about 0.4 millimetres to about 0.8 millimetres.

As illustrated in FIG. 8A, the heater 80 may have a square-shaped base wherein a corner of the heater 80 is arranged to contact the wick 28.

As illustrated in FIG. 8B, the heater 80 may have a triangular-shaped base wherein a corner of the heater 80 is arranged to contact the wick 28.

In at least one example embodiment, the heater 80 contacts the wick 28 such that boundaries 88 are formed there between. The boundaries 88, as shown in FIGS. 8A and 8B, are the portions of the heater 80 that may become wetted with pre-vapor formulation, which may be vaporized by the heater 80. Therefore, by placing the heater 80 in contact with the wick 28, vapor may be formed from the pre-vapor formulation vaporized at the boundary 88 thereof when the patterned layer of platinum 81 is supplied power by the power source (not shown).

FIGS. 10A-10D each illustrates an example embodiment of the heater 80, which may be included in the electronic vaping device 10. In some example embodiments, as shown in FIGS. 10A-10D, the heater 80 includes the patterned layer of platinum 81 disposed on a ceramic layer 82 of material.

As shown in FIGS. 10A and 10B, a glass layer 84 of material may be disposed on the ceramic layer 82 wherein the patterned layer of platinum 81 is between the ceramic layer 82 and the glass layer 84.

In another example embodiment, the ceramic layer 82 is a first ceramic layer, and a second ceramic layer is disposed on the first ceramic layer, such that the patterned layer of platinum 81 is between the first ceramic layer and the second ceramic layer. The leads 83 are electrically connected to the patterned layer of platinum 81, such that the patterned layer of platinum 81 may be electrically connected to the power supply 26.

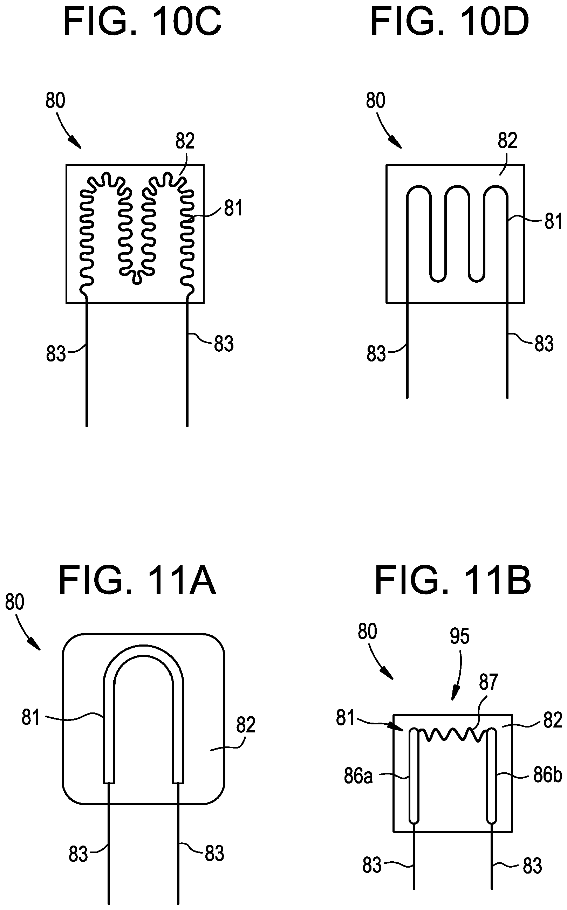

In at least one example embodiment, as shown in FIG. 10A, 10C, and 10D, the patterned layer of platinum 81 may have a sinuous pattern. By increasing the number of turns of the sinuous pattern, and by reducing the spacing between turns of the sinuous pattern, the resistance of the patterned layer of platinum 81 may be increased. Therefore, for the same material, the patterned layers of platinum 81, as shown in FIGS. 10C and 10D, will have a greater resistance than the patterned layer of platinum 81 as shown in FIG. 10A because the patterned layers as shown in FIGS. 10C and 10D have closer spacing and more turns than the patterned layer as shown in FIG. 10A.

FIGS. 11A-11D each illustrates an example embodiment of the heater 80, which may be included in an electronic vaping device 10.

As shown in FIGS. 11A-11D, the patterned layer of platinum 81 may be disposed on the ceramic layer 82 in a generally U-shaped pattern, and the electrical leads 83 are electrically connected to the patterned layer of platinum 81.

As illustrated in FIG. 11A, the patterned layer of platinum 81 is generally U-shaped and the patterned layer of platinum 81 is disposed on ceramic layer 82 so as to evenly heat the heater 80 when power is supplied to the patterned layer of platinum 81 by the power source.

In at least one example embodiment, the patterned layer of platinum 81 may be arranged so as to control the portion of the heater 80, which generates the greatest amount of heat. By controlling the portion of the heater 80 which generates the greatest amount of heat, the heater 80 may be arranged to contact or partially contact the wick 28 at the portion of the heater 80 which generates the greatest amount of heat. Therefore, the portion of the heater 80 which generates the greatest amount of heat may be arranged to be the portion of the heater 80 which becomes wetted by pre-vapor formulation delivered thereto by the wick. In this manner, the power required to vaporize the pre-vapor formulation delivered to the heater 80 may be reduced, the voltage across the patterned layer of platinum required to sufficiently heat the patterned layer of platinum 81 may be reduced, or the length of time that power is supplied to the patterned layer of platinum 81 may be reduced.

In one example embodiment, as illustrated in FIG. 11B, the patterned layer of platinum 81 may be generally U-shaped. The U-shaped layer of platinum 81 includes first and second conductor portions 86a, 86b, and a heater portion 87 extending between the first and second conductor portions 86a, 86b along an upper edge 95 of the heater 80. Since the conductor portions 86a, 86b have a lower resistivity than the heater portion 87, power may be supplied to the patterned layer of platinum 81 such that a greater amount of heat is generated along the upper edge 95 of the heater 80 than the remainder of the heater 80. Therefore, the upper edge 95 of the heater 80 may be arranged to contact the wick wherein less power is required to vaporize pre-vapor formulation along the upper edge 95 of the heater 80 than if the heater 80 were to be evenly heated. In an example embodiment, the conductor portions 86a, 86b may have a thickness of about 20 microns and the heater portion 87 may have a thickness of about 0.5 micron to about 2 microns. The conductor portions 86a, 86b and the heater portion 87 may each have a width of about 1 micron to about 100 microns.

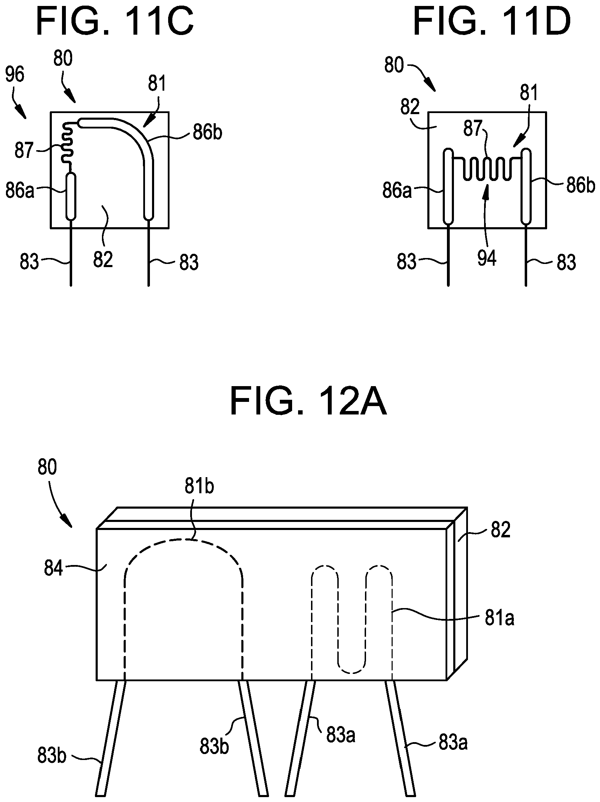

In some example embodiments, as illustrated in FIG. 11C, the heater portion 87 may extend between the first and second conductor portions 86a, 86b along a corner 96 of the heater 80. The heater portion 87 has a higher resistance than the first and second conductor portions 86a, 86b. Power may be supplied to the patterned layer of platinum 81, such that the greatest amount of heat is generated at a corner 96 of the heater 80. Therefore, the corner 96 of the heater 80 may be arranged to contact the wick 28 wherein less power is required to vaporize pre-vapor formulation at the corner 96 of the heater 80 than if the heater 80 were to be evenly heated.

As illustrated in FIG. 11D, in another example embodiment, the heater portion 87 may extend between the first and second conductor portions 86a, 86b at a central region 94 of the heater 80 wherein the heater portion 87 has a higher resistance than the first and second conductor portions 86a, 86b. The greatest amount of heat is generated at the central region 94 of the heater 80. Therefore, the wick 28 may be arranged to extend across the central region 94 of the heater 80 wherein less power is required to vaporize pre-vapor formulation at the central region 94 of the heater 80 than if the heater 80 were to be evenly heated.

FIGS. 12A-12B each illustrates an example embodiment of a heater 80, which may be included in an electronic vaping device 10.

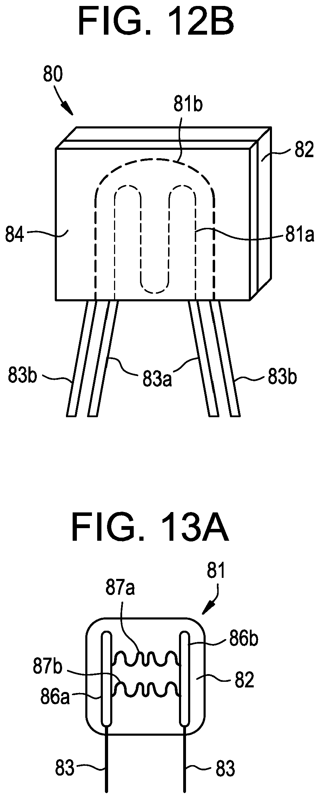

As shown in FIGS. 12A-12B, the heater 80 includes a first patterned layer of platinum 81a disposed on a ceramic layer 82 of material and a second patterned layer of platinum 81b disposed on the ceramic layer 82. The first patterned layer 81a and the second patterned layer 81b may be side by side as shown in FIG. 12A. In at least one example embodiment, as shown in FIG. 12B, the first patterned layer 81a may be nested within the second patterned layer 81b. A glass layer 84 of material may be disposed on the ceramic layer 82. The first and second patterned layers of platinum 81a, 81b may be between the ceramic layer 82 and the glass layer 82. Alternatively, the glass layer 84 may be formed from a ceramic material as opposed to a glass material. Leads 83a are electrically connected to the first patterned layer of platinum 81a such that the first patterned layer of platinum 81a may be electrically connected to a power source (not shown). Leads 83b are electrically connected to the second patterned layer of platinum 81b such that the patterned layer of platinum 81b may be electrically connected to the power supply. The first patterned layer of platinum 81a may have a lower room temperature resistance than the second patterned layer of platinum 81b, such that when power is supplied from the power source to the first and second patterned layers of platinum 81a, 81b, the first patterned layer of platinum 81a may cause the heater 80 to quickly rise in temperature while the second patterned layer of platinum 81b may cause the heater 80 to achieve higher overall temperatures.

FIGS. 13A-13B each illustrates an example embodiment of a heater 80 which may be included in an electronic vaping device 10 as disclosed herein.

As shown in FIG. 13A, the patterned layer of platinum 81 includes first and second conductor portions 86a, 86b and a first heater portion 87a and a second heater portion 87b arranged in parallel between the first and second conductor portions 86a, 86b.

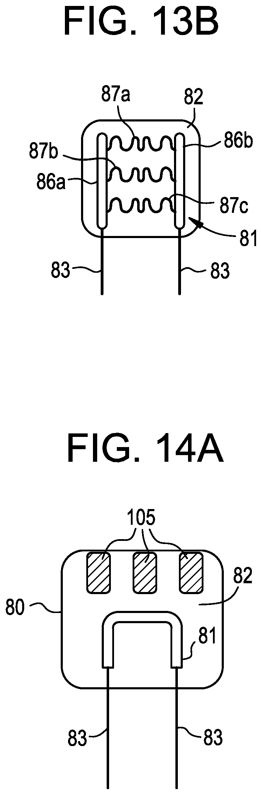

As shown in FIG.13B, the patterned layer of platinum 81 includes first and second conductor portions 86a,b and a first heater portion 87a, a second heater portion 87b, and a third heater portion 87c arranged in parallel between the first and second conductor portions 86a, 86b. In alternate embodiments, more than three heater portions may be arranged in parallel between the first and second conductors 86a, 86b.

By arranging the heater portions in parallel, heat generation may be controlled such that portions of the heater 80 which become wetted by pre-vapor formulation drawn there toward are heated faster than surrounding portions of the heater. For example, if a portion of the heater 80 overlying the first heater portion 87a becomes wetted by pre-vapor formulation, the thermal load of the pre-vapor formulation will cause a drop in resistivity of the first heater portion 87a. As the resistance of the first heater portion 87a drops, more power will be supplied to the first heater portion 87a, thereby causing the first heater portion 87a to increase in temperature and therefore increase the rate of vaporization at the portion of the heater 80 overlying the first heater portion 87a. In this manner, the heater 80 may direct heat to portions thereof with greater thermal load thereby increasing the efficiency of vaporization of pre-vapor formulation delivered thereto.

Referring to FIGS. 14A-14C, the ceramic layer of material 82 may include one or more grooves 105, bumps 106, through-holes 107, and combinations thereof, which are arranged to direct a flow of pre-vapor formulation from the wick toward a portion of the heater 80 that is arranged to reach a temperature sufficient to vaporize the pre-vapor formulation drawn there toward when the patterned layer of platinum is resistively heated.

In some example embodiments, as shown in FIG. 14A, one or more grooves 105 may be arranged to direct the flow of the pre-vapor formulation over a surface of the heater 80 wherein the pre-vapor formulation may fill the grooves 105 and flow toward a portion of the heater 80 that is arranged to reach a temperature to vaporize the pre-vapor formulation and then be vaporized upon reaching that portion.

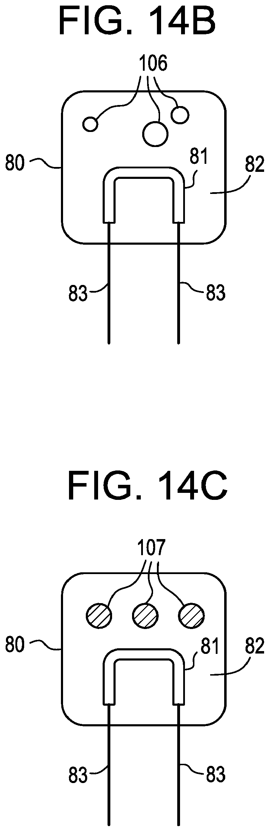

In another example embodiment, as shown in FIG. 14B, one or more bumps 106 which are arranged to direct the flow of pre-vapor formulation over a surface of the heater 80 to reach a temperature sufficient to vaporize the pre-vapor formulation drawn there toward when the patterned layer of platinum is resistively heated.

In at least one embodiment, as shown in FIG. 14C, the ceramic layer of material 82 may include through-holes 107, which are arranged to extend through the ceramic layer of material 82. The through-holes 107 may optionally expose portions of the patterned layer of platinum and wherein the through-holes 107 are arranged to direct the flow of pre-vapor formulation over a surface of the heater 80 wherein the pre-vapor formulation may enter a through hole 107 and thereby be vaporized by the patterned layer of platinum 81 when the patterned layer of platinum is heated.

In some example embodiments, the heater 80 may be a magnetic heater as described in U.S. non-provisional application no. 14/882,665 filed October 15, 2015.

In other example embodiments, the heater 80 may be any heater that is configured to vaporize a pre-vapor formulation without being intertwined with a wick. Therefore, the heater 80 may be any planar heater.

In at least one example embodiment, the heater may be a thin film ceramic heater including a thin film of an oxidation resistant conductor on a ceramic, such as alumina in contact with a wick.

In at least one example embodiment, the heater may include a thin film ceramic heater shaped like a cylinder or tube.

In at least one example embodiment, the heater may be a nickel-chromium wire wrapped around a ceramic cylinder, tube, disc, square, or rectangle. In this example embodiment, the heater may be supported by leads.

In at least one example embodiment, the heater may be a nickel-chromium wire wrapped around a ceramic or glass wick. In this example embodiment, the heater may be supported by leads.

In at least one example embodiment, the electrical resistance of the heater is about 2 to about 10 ohms. In at least one example embodiment, the maximum linear dimension of the heater ranges from about 5 millimetres to about 10 millimetres and the volume ranges from about 1 cubic millimetres to about 10 cubic millimetres.

In an example embodiment, the electronic vaping device 10 may be about 80 millimetres to about 110 millimetres long and about 7 millimetres to about 8 millimetres in diameter. For example, in one example embodiment, the e-vaping device may be about 84 millimetres long and may have a diameter of about 7.8 millimetres.

While a number of example embodiments have been disclosed herein, it should be understood that other variations may be possible. Such variations are not to be regarded as a departure from the scope of the present disclosure, and all such modifications as would be obvious to one skilled in the art are intended to be included within the scope of the following claims.

## Claims

1. An electronic vaping device (10) comprising:
a housing (32) extending in a longitudinal direction, the housing (32) having a tip end (14) and a mouth-end (12), the tip end (14) being closed and the mouth-end (12) having an opening (5) therein;
a planar heater (80) contained in the housing (32);
a heater support (24) configured to support the planar heater (80), the heater support (24) comprising:
a disc-shaped body (25) that friction fits with an inner surface (102) of the housing (32), and
a tubular body (21) on which the heater (80) is mounted, the tubular body (21) extending downstream from the disc-shaped body (25);
a tank (16) containing a pre-vapor formulation, the tank (16) configured to slide into and out of the opening (5) of the mouth-end (12) of the housing (32); and
a wick (28) extending from the tank (16), the wick (28) configured to be in contact with the planar heater (80) when the tank (16) is inserted in the housing (32).

2. The electronic vaping device (10) of claim 1, further comprising:
a mouth-end insert (8) configured to be inserted in the mouth-end (12) of the housing (32), the mouth-end insert (8) including at least one outlet.

3. The electronic vaping device (10) of claim 1 or 2, further comprising:
a stop (36) on an inner surface (102) of the housing (32), the stop (36) configured to substantially prevent the tank (16) from being inserted too far into the housing (32).

4. The electronic vaping device (10) of claim 1, 2 or 3, wherein the housing (32) is unitary; and/or optionally
wherein the wick (28) is formed of cellulose; and/or optionally
wherein the wick (28) is monolithic; and/or optionally
wherein the tank (16) includes one or more ribs (18) running longitudinally along an outer surface (110) of the tank (16).

5. The electronic vaping device (10) of any preceding claim, wherein the planar heater (80) comprises:
a patterned layer of platinum (81) disposed on a ceramic layer (82), the patterned layer of platinum (81) being configured to be in electrical communication with a power supply (26) through leads (83) electrically connected to the patterned layer of platinum (81).

6. The electronic vaping device (10) of claim 5, wherein the power supply (26) is configured to supply power to the patterned layer of platinum (81) so as to resistively heat the patterned layer of platinum (81) such that the heater (80) may reach a temperature sufficient to vaporize the pre-vapor formulation.

7. The electronic vaping device (10) of claim 5 or 6, wherein the patterned layer of platinum (81) has a resistivity of about 1 to 6 ohms; and/or optionally
wherein the leads (83) are formed from platinum coated nickel wire; and/or optionally
wherein the heater (80) is in the shape of a polyhedron having a square, triangular, diamond or rectangular shaped base with rounded or sharp corners; and/or optionally
wherein the heater (80) has a square or rectangular base wherein a length and width of the heater are each about 1.5 to 4 millimetres and a thickness of the heater is about 0.2 to 0.8 millimetres.

8. The electronic vaping device (10) of any of claims 5 to 7, wherein a glass layer (84) is disposed on the ceramic layer (82) such that the patterned layer of platinum (81) is between the ceramic layer (82) and the glass layer (84).

9. The electronic vaping device (10) of any of claims 5 to 7, wherein the ceramic layer (82) is a first ceramic layer, and a second ceramic layer is disposed on the first ceramic layer such that the patterned layer of platinum (81) is between the first ceramic layer and the second ceramic layer.

10. The electronic vaping device (10) of any of claims 5 to 9, wherein the ceramic layer (82) is formed from alumina, titania, zirconia, yttria, or yttria-stabilized zirconia; and/or optionally
wherein the patterned layer of platinum (81) is about 0.5 micron to about 2 microns thick and has a width ranging from about 1 micron to about 100 microns; and/or optionally
wherein the patterned layer of platinum (81) has a sinuous pattern; and/or optionally
wherein the patterned layer of platinum (81) has a U-shaped pattern; and/or optionally
wherein the patterned layer of platinum (81) comprises:
a first conductor (86a), a second conductor (86b), and at least two heater portions (87a, 87b) arranged in parallel between the first (86a) and second (86b) conductors, wherein the heater portions (87a, 87b) have a higher resistivity than the first (86a) and second (86b) conductors.

11. The electronic vaping device (10) of any of claims 5 to 10, wherein the heater (80) comprises:
a first patterned layer of platinum (81a) and a second patterned layer of platinum (81b), the first patterned layer of platinum (81a) having a higher resistivity than the second patterned layer of platinum (81b), the first patterned layer of platinum (81a) being configured to be in electrical communication with the power source (26) through a first set of leads (83a) and the second layer of platinum (81b) being configured to be in electrical communication with the power source (26) through a second set of leads (83b).

12. The electronic vaping device (10) of claim 11, wherein the first patterned layer of platinum (81a) is sinuous and the second patterned layer of platinum (81b) is U-shaped.

13. The electronic vaping device (10) of any of claims 5 to 12, wherein the ceramic layer (82) comprises:
at least one groove (105) in a surface thereof, the groove (105) is configured to direct a flow of the pre-vapor formulation from the wick (28) toward a portion of the heater (80) that reaches a temperature sufficient to vaporize pre-vapor formulation and/or optionally
wherein the ceramic layer (82) comprises:
at least one through-hole (107) extending through a thickness of the ceramic layer (82), the at least one through-hole (107) exposing portions of the patterned layer of platinum (81), the through-hole (107) being configured to direct a flow of the pre-vapor formulation from the wick (28) toward a portion of the heater (80); and/or optionally
wherein the ceramic layer (82) is porous; and/or optionally
wherein the ceramic layer (82) comprises:
at least one bump (106), the bump (106) configured to direct a flow of the pre-vapor formulation from the wick (28) toward a portion of the heater (80).

14. The electronic vaping device (10) of any of claims 5 to 13, wherein the patterned layer of platinum (81) includes first (86a) and second (86b) conductors and a heater portion (87) arranged between the first (86a) and second (86b) conductors, the first (86a) and second (86b) conductors each having a thickness of about 20 microns, and the heater portion (87) having a thickness of about 2 microns; and/or optionally
wherein the patterned layer of platinum (81) includes a gold coating on an outer surface thereof; and/or optionally
wherein the patterned layer of platinum (81) is configured to concentrate heat at a tip thereof, and the tip of the heater (80) is thermally isolated from the remainder of the heater (80).

15. The electronic vaping device (10) of any preceding claim, wherein the electronic vaping device (10) has a uniform diameter of less than about 10 millimetres; and/or optionally
wherein the electronic vaping device (10) further comprises:
control circuitry (20) including a sensor (3), the sensor (3) configured to sense a change in pressure; and
at least one light emitting diode (30) at the tip end (14).

## Patentansprüche

1. Elektronische Dampfvorrichtung (10), aufweisend:
ein Gehäuse (32), das sich in einer Längsrichtung erstreckt, wobei das Gehäuse (32) ein Endstück (14) und ein Mundende (12) aufweist, das Endstückende (14) geschlossen ist und das Mundende (12) eine Öffnung (5) darin aufweist;
eine planare Heizvorrichtung (80), die in dem Gehäuse (32) enthalten ist;
eine Heizvorrichtungsauflage (24), die ausgelegt ist, die planare Heizvorrichtung (80) zu tragen, wobei die Heizvorrichtungsauflage (24) aufweist:
einen scheibenförmigen Körper (25), der mit einer Innenfläche (102) des Gehäuses (32) eine Friktionspassung bildet, und
einen rohrförmigen Körper (21), auf dem die Heizvorrichtung (80) angebracht ist, wobei sich der rohrförmige Körper (21) nachgeschaltet des scheibenförmigen Körpers (25) erstreckt;
einen Behälter (16), der eine Vordampfformulierung enthält, wobei der Behälter (16) ausgelegt ist, in die Öffnung (5) des Mundendes (12) des Gehäuses (32) und daraus heraus zu gleiten; und
einen Docht (28), der sich von dem Behälter (16) erstreckt, wobei der Docht (28) ausgelegt ist, in Kontakt mit der planaren Heizvorrichtung (80) zu sein, wenn der Behälter (16) in das Gehäuse (32) eingesetzt ist.

2. Elektronische Dampfvorrichtung (10) nach Anspruch 1, weiter aufweisend:
einen Mundendeeinsatz (8), der ausgelegt ist, in das Mundende (12) des Gehäuses (32) eingesetzt zu werden, wobei der Mundendeeinsatz (8) mindestens einen Auslass einschließt.

3. Elektronische Dampfvorrichtung (10) nach Anspruch 1 oder 2, weiter aufweisend:
einen Anschlag (36) an einer Innenfläche (102) des Gehäuses (32), wobei der Anschlag (36) ausgelegt ist, im Wesentlichen zu verhindern, dass der Behälter (16) zu weit in das Gehäuse (32) eingesetzt wird.

4. Elektronische Dampfvorrichtung (10) nach Anspruch 1, 2 oder 3, wobei das Gehäuse (32) einheitlich ist; und/oder optional
wobei der Docht (28) aus Zellulose gebildet ist; und/oder optional
wobei der Docht (28) monolithisch ist; und/oder optional
wobei der Behälter (16) eine oder mehrere Rippen (18) einschließt, die in Längsrichtung entlang einer Außenfläche (110) des Behälters (16) verlaufen.

5. Elektronische Dampfvorrichtung (10) nach einem der vorstehenden Ansprüche, wobei die planare Heizvorrichtung (80) aufweist:
eine strukturierte Schicht aus Platin (81), die auf einer keramischen Schicht (82) angeordnet ist, wobei die strukturierte Schicht aus Platin (81) ausgelegt ist, durch Leitungen (83) in elektrischer Verbindung mit einer Stromversorgung (26) zu sein, die mit der gemusterten Schicht aus Platin (81) elektrisch verbunden ist.

6. Elektronische Dampfvorrichtung (10) nach Anspruch 5, wobei die Stromversorgung (26) zum Bereitstellen von Strom an die strukturierte Schicht aus Platin (81) ausgelegt ist, sodass sie die strukturierte Schicht aus Platin (81) derart erwärmt, dass die Heizvorrichtung (80) eine Temperatur erreichen kann, die ausreichend ist, um die Vordampfformulierung zu verdampfen.

7. Elektronische Dampfvorrichtung (10) nach Anspruch 5 oder 6, wobei die strukturierte Schicht aus Platin (81) einen spezifischen Widerstand von ungefähr 1 bis 6 Ohm aufweist; und/oder optional
wobei die Leitungen (83) aus mit Platin beschichtetem Nickeldraht gebildet sind; und/oder optional
wobei die Heizvorrichtung (80) die Form eines Polyeders mit einer quadratisch, dreieckig, rautenförmig oder rechteckig geformten Basis mit abgerundeten oder scharfen Ecken aufweist; und/oder optional
wobei die Heizvorrichtung (80) eine quadratische oder rechteckige Basis aufweist, wobei eine Länge und Breite der Heizvorrichtung jeweils ungefähr 1,5 bis 4 Millimeter beträgt und eine Dicke der Heizvorrichtung ungefähr 0,2 bis 0,8 Millimeter beträgt.

8. Elektronische Dampfvorrichtung (10) nach einem der Ansprüche 5 bis 7, wobei eine Glasschicht (84) auf der keramischen Schicht (82) derart angeordnet ist, dass sich die strukturierte Schicht aus Platin (81) zwischen der keramischen Schicht (82) und der Glasschicht (84) befindet.

9. Elektronische Dampfvorrichtung (10) nach einem der Ansprüche 5 bis 7, wobei die keramische Schicht (82) eine erste keramische Schicht ist und eine zweite keramische Schicht auf der ersten keramischen Schicht derart angeordnet ist, dass sich die strukturierte Schicht aus Platin (81) zwischen der ersten keramischen Schicht und der zweiten keramischen Schicht befindet.

10. Elektronische Dampfvorrichtung (10) nach einem der Ansprüche 5 bis 9, wobei die keramische Schicht (82) aus Aluminiumoxid, Titandioxid, Zirkondioxid, Yttriumoxid oder yttriumoxidstabilisiertem Zirkondioxid gebildet ist; und/oder optional
wobei die strukturierte Schicht aus Platin (81) ungefähr 0,5 Mikrometer bis zu ungefähr 2 Mikrometer dick ist und eine Breite im Bereich von ungefähr 1 Mikrometer bis zu ungefähr 100 Mikrometer aufweist; und/oder optional
wobei die strukturierte Schicht aus Platin (81) ein kurviges Muster aufweist; und/oder optional
wobei die gemusterte Schicht aus Platin (81) ein U-förmiges Muster aufweist; und/oder optional
wobei die gemusterte Schicht aus Platin (81) aufweist:
einen ersten Leiter (86a), einen zweiten Leiter (86b) und mindestens zwei Heizvorrichtungsabschnitte (87a, 87b), die zwischen den ersten (86a) und zweiten (86b) Leitern parallel angeordnet sind, wobei die Heizvorrichtungsabschnitte (87a, 87b) einen höheren spezifischen Widerstand aufweisen als die ersten (86a) und zweiten (86b) Leiter.

11. Elektronische Dampfvorrichtung (10) nach einem der Ansprüche 5 bis 10, wobei die Heizvorrichtung (80) aufweist:
eine erste strukturierte Schicht aus Platin (81a) und eine zweite strukturierte Schicht aus Platin (81b), wobei die erste strukturierte Schicht aus Platin (81a) einen höheren spezifischen Widerstand aufweist als die zweite strukturierte Schicht aus Platin (81b), die erste strukturierte Schicht aus Platin (81a) ausgelegt ist, durch einen ersten Satz von Leitungen (83a) in elektrischer Verbindung mit der Stromquelle (26) zu sein, und die zweite Schicht aus Platin (81b) ausgelegt ist, durch einen zweiten Satz von Leitungen (83b) in elektrischer Verbindung mit der Stromquelle (26) zu sein.

12. Elektronische Dampfvorrichtung (10) nach Anspruch 11, wobei die erste strukturierte Schicht aus Platin (81a) kurvig ist und die zweite strukturierte Schicht aus Platin (81b) U-förmig ist.

13. Elektronische Dampfvorrichtung (10) nach einem der Ansprüche 5 bis 12, wobei die keramische Schicht (82) aufweist:
mindestens eine Nut (105) in einer Fläche davon, wobei die Nut (105) ausgelegt ist, eine Strömung der Vordampfformulierung von dem Docht (28) zu einem Abschnitt der Heizvorrichtung (80) zu lenken, der eine Temperatur erreicht, die ausreichend ist, um eine Vordampfformulierung zu verdampfen, und/oder optional
wobei die keramische Schicht (82) aufweist:
mindestens eine Durchgangsbohrung (107), die sich durch eine Dicke der keramischen Schicht (82) erstreckt, wobei die mindestens eine Durchgangsbohrung (107) Abschnitte der gemusterten Schicht aus Platin (81) freilegt und die Durchgangsbohrung (107) ausgelegt ist, eine Strömung der Vordampfformulierung von dem Docht (28) zu einem Abschnitt der Heizvorrichtung (80) zu lenken; und/oder optional
wobei die keramische Schicht (82) porös ist; und/oder optional
wobei die keramische Schicht (82) aufweist:
mindestens einen Höcker (106), wobei der Höcker (106) ausgelegt ist, eine Strömung der Vordampfformulierung von dem Docht (28) zu einem Abschnitt der Heizvorrichtung (80) zu lenken.

14. Elektronische Dampfvorrichtung (10) nach einem der Ansprüche 5 bis 13, wobei die strukturierte Schicht aus Platin (81) erste (86a) und zweite (86b) Leiter und einen Heizvorrichtungsabschnitt (87) aufweist, der zwischen den ersten (86a) und zweiten (86b) Leitern angeordnet ist, die ersten (86a) und zweiten (86b) Leiter jeweils eine Dicke von ungefähr 20 Mikrometer aufweisen und der Heizvorrichtungsabschnitt (87) eine Dicke von ungefähr 2 Mikrometer aufweist; und/oder optional
wobei die strukturierte Schicht aus Platin (81) eine Goldschicht auf einer Außenfläche davon einschließt; und/oder optional
wobei die strukturierte Schicht aus Platin (81) ausgelegt ist, Wärme an einem Endstück davon zu konzentrieren und das Endstück der Heizvorrichtung (80) von dem Rest der Heizvorrichtung (80) wärmeisoliert ist.

15. Elektronische Dampfvorrichtung (10) nach einem der vorstehenden Ansprüche, wobei die elektronische Dampfvorrichtung (10) einen einheitlichen Durchmesser von kleiner als ungefähr 10 Millimetern aufweist; und/oder optional
wobei die elektronische Dampfvorrichtung (10) weiter aufweist:
Steuerschaltungen (20) einschließlich eines Sensors (3), wobei der Sensor (3) ausgelegt ist, eine Druckänderung zu erfassen; und
mindestens eine Leuchtdiode (30) an dem Endstückende (14).

## Revendications

1. Dispositif de vapotage électronique (10), comprenant :
un boîtier (32) s'étendant dans une direction
longitudinale, le boîtier (32) ayant une extrémité de pointe (14) et une extrémité buccale (12), l'extrémité de pointe (14) fermée et l'extrémité buccale (12) ayant une ouverture (5) dans celle-ci ;
un dispositif de chauffage plat (80) contenu dans le boîtier (32) ;
un support de chauffage (24) configuré pour soutenir le dispositif de chauffage plat (80), le support de chauffage (24) comprenant :
un corps en forme de disque (25) qui s'ajuste par frottement à une surface intérieure (102) du boîtier (32), et
un corps tubulaire (21) sur lequel le dispositif de chauffage (80) est monté, le corps tubulaire (21) s'étendant en aval du corps en forme de disque (25) ;
un réservoir (16) contenant une formulation pré-vapeur, le réservoir (16) configuré pour glisser dans et hors de l'ouverture (5) de l'extrémité buccale (12) du boîtier (32) ; et
une mèche (28) s'étendant du réservoir (16), la mèche (28) configurée pour être en contact avec le dispositif de chauffage plat (80) lorsque le réservoir (16) est inséré dans le boîtier (32).

2. Dispositif de vapotage électronique (10) selon la revendication 1, comprenant en outre :
une insertion d'extrémité buccale (8) configurée pour être insérée dans l'extrémité buccale (12) du boîtier (32), insertion d'extrémité buccale (8) incluant au moins une sortie.

3. Dispositif de vapotage électronique (10) selon la revendication 1 ou 2, comprenant en outre :
un butée (36) sur une surface intérieure (102) du boîtier (32), la butée (36) étant configuré pour empêcher substantiellement le réservoir (16) d'être inséré trop loin dans le boîtier (32).

4. Dispositif de vapotage électronique (10) selon la revendication 1, 2 ou 3, dans lequel le boîtier (32) est unitaire ; et/ou facultativement
dans lequel la mèche (28) est formée de cellulose ; et/ou facultativement
dans lequel la mèche (28) est monolithique ; et/ou facultativement
dans lequel le réservoir (16) comprend une ou plusieurs nervures (18) fonctionnant longitudinalement le long d'une surface extérieure (110) du réservoir (16).

5. Dispositif de vapotage électronique (10) selon l'une quelconque revendication précédente, dans lequel le dispositif de chauffage (80) comprend :
une couche à motifs de platine (81) disposée sur une couche en céramique (82), la couche à motifs de platine (81) étant configurée pour être en communication électrique avec une alimentation électrique (26) à travers les conducteurs (83) reliée électriquement à la couche à motifs de platine (81) .

6. Dispositif de vapotage électronique (10) selon la revendication 5, dans lequel l'alimentation électrique (26) est configurée pour alimenter l'alimentation à la couche à motifs de platine (81) afin de chauffer de manière résistive la couche à motifs de platine (81) de sorte que le dispositif de chauffage (80) puisse atteindre une température suffisante pour vaporiser la formulation pré-vapeur.

7. Dispositif de vapotage électronique (10) selon la revendication 5 ou 6, dans lequel la couche à motifs de platine (81) a une résistivité d'environ 1 à 6 ohms ; et/ou facultativement
dans lequel les conducteurs (83) sont formés à partir de fils de nickel recouverts de platine ; et/ou facultativement
dans lequel le dispositif de chauffage (80) est en forme d'un polyèdre ayant une base carrée, triangulaire, en forme de diamant ou rectangulaire avec des coins arrondis ou coupants ; et/ou facultativement
dans lequel le dispositif de chauffage (80) possède une base carrée ou rectangulaire, dans lequel une longueur et une largeur du dispositif de chauffage sont chacune d'environ 1,5 à 4 millimètres et une épaisseur du chauffage est d'environ 0,2 à 0,8 millimètres.

8. Dispositif de vapotage électronique (10) selon l'une quelconque des revendications 5 à 7, dans lequel une couche en verre (84) est disposée sur la couche céramique (82) de telle sorte que la couche à motifs de platine (81) se trouve entre la couche en céramique (82) et la couche en verre (84).

9. Dispositif de vapotage électronique (10) selon l'une quelconque des revendications 5 à 7, dans lequel la couche céramique (82) est une première couche céramique, et une deuxième couche céramique est disposée sur la première couche céramique de sorte que la couche à motifs de platine (81) se trouve entre la première couche céramique et la deuxième couche céramique.

10. Dispositif de vapotage électronique (10) selon l'une quelconque des revendications 5 à 9, dans lequel la couche céramique (82) est formée à partir d'alumine, de titane, de zircone, d'yttrium ou de zircone stabilisée par l'yttrium ; et/ou facultativement
dans lequel la couche à motifs de platine (81) est d'environ 0,5 micron à environ 2 microns d'épaisseur et a une largeur allant d'environ 1 micron à environ 100 microns ; et/ou facultativement
dans lequel la couche à motifs de platine (81) a un motif sinueux ; et/ou facultativement
dans lequel la couche à motifs de platine (81) a un motif en forme de U ; et/ou facultativement
dans lequel la couche à motifs de platine (81) comprend :
un premier conducteur (86a), un deuxième conducteur (86b), et au moins deux parties chauffantes (87a, 87b) agencées en parallèle entre les premiers (86a) et seconds (86b) conducteurs, dans lequel les portions de chauffage (87a, 87b) ont une résistivité supérieure aux premiers (86a) et seconds (86b) conducteurs.

11. Dispositif de vapotage électronique (10) selon l'une quelconque des revendications 5 à 10, dans lequel le dispositif de chauffage (80) comprend :
une première couche à motifs de platine (81a) et une deuxième couche à motifs de platine (81b), la première couche à motifs de platine (81a) ayant une résistivité supérieure à la deuxième couche à motifs de platine (81b), la première couche à motifs de platine (81a) étant configurée pour être en communication électrique avec la source d'alimentation (26) à travers un premier ensemble de conducteurs (83a) et la deuxième couche de platine (81b) étant configurée pour être en communication électrique avec la source d'alimentation (26) à travers un deuxième jeu de conducteurs (83b).

12. Dispositif de vapotage électronique (10) selon la revendication 11, dans lequel la première couche à motifs de platine (81a) est sinueuse et la deuxième couche à motifs de platine (81b) est en forme de U.

13. Dispositif de vapotage électronique (10) selon l'une quelconque des revendications 5 à 12, dans lequel la couche céramique (82) comprend :
au moins une rainure (105) sur une surface de celle-ci, la rainure (105) est configurée pour diriger un flux de la formule pré-vapeur de la mèche (28) vers une partie du dispositif de chauffage (80) qui atteint une température suffisante pour vaporiser la formule pré-vapeur et/ou facultativement
dans lequel la couche céramique (82) comprend :
au moins un trou traversant (107) s'étendant à travers une épaisseur de la couche céramique (82), l'au moins un trou traversant (107) exposant des parties de la couche de platine (81), le trou traversant (107) étant configuré pour diriger un flux de la formule pré-vapeur de la mèche (28) vers une partie du dispositif de chauffage (80) ; et/ou facultativement
dans lequel la couche céramique (82) est poreuse ; et/ou facultativement
dans lequel la couche céramique (82) comprend :
au moins une protubérance (106), la protubérance (106) configurée pour diriger un flux de la formule pré-vapeur de la mèche (28) vers une partie du dispositif de chauffage (80) .

14. Dispositif de vapotage électronique (10) selon l'une quelconque des revendications 5 à 13, dans lequel la couche à motifs de platine (81) comprend des premiers (86a) et seconds (86b) conducteurs et une partie de chauffage (87) disposés entre les premiers (86a) et seconds (86b) conducteurs, les premiers (86a) et seconds (86b) conducteurs ayant chacun une épaisseur d'environ 20 microns, et la partie du chauffage (87) ayant une épaisseur d'environ 2 microns ; et/ou facultativement
dans lequel la couche à motifs de platine (81) comprend un revêtement doré sur une surface extérieure de celui-ci ; et/ou facultativement
dans lequel la couche à motifs de platine (81) est configurée pour concentrer la chaleur à une extrémité de celle-ci, et l'extrémité du dispositif de chauffage (80) est isolée thermiquement du reste du dispositif de chauffage (80) .

15. Dispositif de vapotage électronique (10) selon une quelconque revendication précédente, dans lequel le dispositif de vapotage électronique (10) a un diamètre uniforme inférieur à environ 10 millimètres ; et/ou facultativement
dans lequel le dispositif de vapotage électronique (10) comprend en outre :
un circuit de commande (20) comprenant un capteur (3), le capteur (3) configuré pour détecter un changement de pression ; et
au moins une diode électroluminescente (30) à l'extrémité de pointe (14).
